(19) Europäisches Patentamt
European Patent Office
Office européen des brevets

(11) Veröffentlichungsnummer: **0 008 624**
**B1**

(12) # EUROPÄISCHE PATENTSCHRIFT

(45) Veröffentlichungstag der Patentschrift: **24.02.82**

(51) Int. Cl.³: **C 07 D 213/64,**
A 01 N 43/40

(21) Anmeldenummer: **79102185.0**

(22) Anmeldetag: **29.06.79**

(54) **Ester von O-(Pyridyloxy-phenyl)-milchsäuren, Verfahren zu ihrer Herstellung und ihre Verwendung als Herbizide und Pflanzenwachstumsregulatoren.**

(30) Priorität: **03.07.78 CH 7242/78**

(43) Veröffentlichungstag der Anmeldung:
**19.03.80 Patentblatt 80/6**

(45) Bekanntmachung des Hinweises auf die Patenterteilung:
**24.02.82 Patentblatt 82/8**

(84) Benannte Vertragsstaaten:
**AT BE CH DE FR GB IT NL**

(56) Entgegenhaltungen:
**DE - A1 - 2 546 251**
**DE - A1 - 2 628 384**
**DE - A1 - 2 709 108**
**DE - A1 - 2 715 284**
**DE - A1 - 2 732 846**
**DE - A1 - 2 812 571**
**EP - A1 - 0 000 483**

(73) Patentinhaber: **CIBA-GEIGY AG**
**Patentabteilung Postfach**
**CH-4002 Basel (CH)**

(72) Erfinder: **Rempfler, Hermann, Dr.**
**Allschwilerweg 67**
**CH-4102 Binningen (CH)**
Erfinder: **Schurter, Rolf, Dr.**
**Holzmattstrasse 45**
**CH-4102 Binningen (CH)**
Erfinder: **Böhner, Beat, Dr.**
**Hügelweg 3**
**CH-4102 Binningen (CH)**

(74) Vertreter: **Zumstein sen., Fritz, Dr. et al,**
**Bräuhausstrasse 4**
**D-8000 München 2 (DE)**

EP 0 008 624 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Ester von O-(Pyridyloxy-phenyl)-milchsäuren, Verfahren zu ihrer Herstellung und ihre Verwendung als Herbizide und Pflanzenwachstumregulatoren.

Aus dem Stand der Technik ist es bekannt, dass substituierte Phenoxy-propionsäurederivate als Herbizide verendet werden können, vgl. R. Wegler Chemie der Pflanzenschutz- und Schädlingsbekämpfungsmittel, Springer-Verlag Berlin, Heidelberg, New York, 1970, Baud 2, Seiten 278ff. und neuere Patentanmeldungen wie z.B. DOS 2 223 894, DOS 2 613 697, BE 855 094.

Die herbizide Wirksamkeit der bekannten Verbindungen befriedigt aber — insbesondere in tiefen Aufwandmengen — nicht immer. Ausserdem sind solche Verbindungen oft nicht genügend selektiv gegenüber den zu schützenden Kulturpflanzen. Es sind bereits Versuche unternommen worden, die erwähnten Schwächen der bekannten Propionsäure-derivate durch den Einbau geeigneter funktioneller Gruppen im Esterteil des Moleküls zu überwinden. Im Rahmen dieser Arbeiten sind auch substituierte Phenoxy-propionsäurederivate, welche im Esterteil des Moleküls eine zusätzliche Alkoxycarbonyl (COOAlkyl) resp. eine Carboxygruppe (COOH) tragen, bekannt geworden, vgl. DOS 2 628 384.

Pyridyloxy-phenoxy-alkancarbonsäurederivate mit ähnlicher Konstitution aber weniger ausgeprägter Herbizidwirkung sind in der DOS 2 732 846 beschrieben. Pyridyl-2-oxy-alkancarbonsäureester mit Herbizidwirkung sind aus der DOS 2 709 108 bekannt geworden.

Gegenstand vorliegender Erfindung sind neue herbizid und regulierend auf das Pflanzenwachstum einwirkende Ester von O-(Pyridyloxy-phenyl)-milchsäuren, Verfahren zu ihrer Herstellung, Mittel welche diese Ester als Wirkstoffe enthalten, sowie die Verwendung dieser Ester oder sie enthaltender Mittel zur Unkrautbekämpfung und zur Regulierung des Pflanzenwachstums.

Ueberraschenderweise sind die erfindungsgemässen Wirkstoffe der Formel I den bisher bekannt gewordenen, strukturähnlichen Pyridyloxy-phenoxy und Pyridyloxy-alkancarbonsäurederivaten wie auch den Phenoxy-phenoxy-propionsäureestern, welche im Esterteil durch eine zusätzliche Esterfunktion substituiert sind, des Standes der Technik überlegen. Die neuen Wirkstoffe zeichnen sich durch eine hohe Aktivität gegenüber zahlreichen Unkräutern aus. Diese Wirkung ist vor allem gegen die grasartigen, monokotylen Unkräuter ausgeprägt, während Kulturpflanzen wie Soja, Baumwolle und Zuckerrüben, für gewisse Verbindungen auch Weizen weitgehend geschont werden. Diese Verbindungen lassen sich zur selektiven Bekämpfung von Gräsern in diesen Kulturen einsetzen.

Die neuen Wirkstoffe vorliegender Erfindung sind O - [4 - (5' - Halogenpyridyl - 2' - oxy)-phenyl)milchsäureester und entsprechen der Formel I

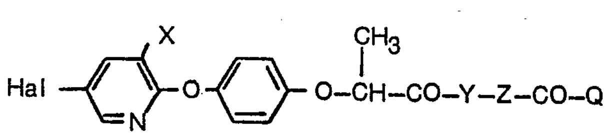

(I)

worin

Hal ein Halogenatom,

X Wasserstoff oder ein Halogenatom,

Y ein Sauerstoff- oder Schwefelatom

Z eine $C_1$—$C_4$ Alkylengruppe, die gegebenenfalls durch Methyl, Aethyl, Cyan, Methylcarbonyl oder eine Gruppe —COQ substituiert sein kann,

Q einen Rest —$OR_1$, —$SR_2$ oder —$NR_3R_4$,

$R_1$ und $R_2$ unabhängig voneinander je

— Wasserstoff oder das Kation-äquivalent einer

$$\frac{1}{n}\,\text{Base—}M^{n\oplus},$$

wobei

M ein n-wertiges Alkali- oder Erdalkalimetall Kation oder ein Fe-, Cu-, Zn-, Mn-, Ni-Kation oder einen Ammonio-Rest

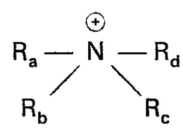

n als ganze Zahl 1, 2 oder 3 der Wertigkeit des Kations entspricht, während $R_a$, $R_b$, $R_c$ und $R_d$ unabhängig voneinander Wasserstoff, Benzyl oder einen gegebenenfalls durch —OH, —$NH_2$ oder $C_1$—$C_4$ Alkoxy substituierten $C_1$—$C_4$ Alkylrest bedeuten.

— einen $C_1$—$C_{18}$ Alkyl-Rest, der unsubstituiert oder gegebenenfalls substituiert ist durch Halogen, Nitro, Cyano, $C_1$—$C_8$ Alkoxy, $C_2$—$C_8$ Alkoxy-alkoxy; $C_3$—$C_6$ Alkenyloxy, $C_1$—$C_8$ Alkylthio, $C_2$—$C_8$ Alkanoyl, $C_2$—$C_8$ Acyloxy, $C_2$—$C_8$ Alkoxycarbonyl, Carbamoyl; Bis ($C_1$—$C_4$ alkyl) amino, Tris-

($C_1$—$C_4$ alkyl)ammonio, $C_3$—$C_8$ Cycloalkenyl, gegebenenfalls auch durch einen unsubstituierten oder seinerseits durch Halogen, $C_1$—$C_4$ Alkyl, $C_1$—$C_4$ Alkoxy ein- oder mehrfach substituierten Phenyl-, Phenoxy- oder 5,6-gliedrigen heterocyclischen Rest mit 1 bis 3 Heteroatomen;

— einen unsubst. oder ein- bis vierfach durch Halogen oder einen durch Phenyl oder Methoxycarbonyl subst. $C_3$—$C_{18}$ Alkenylrest

— einen $C_3$—$C_8$ Alkinyl-Rest

— einen gegebenenfalls durch Halogen oder $C_1$—$C_4$ Alkyl substituierten $C_3$—$C_{12}$ Cycloalkyl-Rest;

— einen $C_3$—$C_8$ Cycloalkenyl-Rest;

— einen Phenylrest, der unsubstituiert oder durch Halogen, $C_1$—$C_4$-Alkyl, $C_1$—$C_4$-Alkoxy, $C_1$—$C_4$-Alkylthio, $NO_2$, $CF_3$, COOH, CN, OH, $SO_3H$, $NH_2$ oder —NH($C_1$—$C_4$ Alkyl) oder —N($C_1$—$C_4$ Alkyl)$_2$ ein- oder mehrfach substituiert ist;

— einen 5- bis 6-gliedrigen heterocyclischen Ring mit 1 bis 3 Heteroatomen

$R_3$ und $R_4$ unabhängig voneinander je Wasserstoff oder $C_1$—$C_4$ Alkyl das gegebenenfalls durch Chlor, Cyan, Hydroxyl oder $C_1$—$C_4$ Alkoxy substituiert ist,

$C_3$—$C_4$ Alkenyl

$R_3$ zudem auch $C_3$—$C_4$ Alkinyl, $C_1$—$C_4$ Alkoxy, Phenyl oder Benzyl, oder

$R_3$ und $R_4$ zusammen mit dem Stickstoffatom, an das sie gebunden sind auch einen 5—6 gliedrigen Heterocyclus bilden, der Sauerstoff, Schwefel oder eine Gruppe

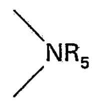

enthalten kann, $R_5$ Wasserstoff, $C_1$—$C_4$ Alkyl oder Phenyl bedeuten.

Die Herstellung der neuen Verbindungen der Formel I erfolgt nach an sich bekannten Reaktionen der chemischen Synthese, z.B. gemäss einem der untenstehenden Schemata:

a)

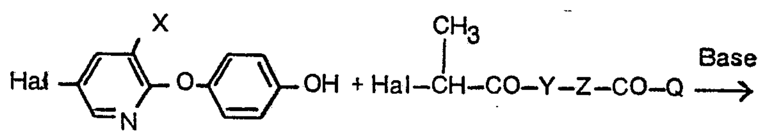


I

Ein entsprechend substituiertes 4 - (5′ - Halogen - pyridyl - 2′ - oxy) - phenol wird in Gegenwart einer Base als säurebindendes Mittel vorteilhafterweise in einem inerten Lösungsmittel mit einem entsprechenden Derivat der 2-Halogenpropionsäure umgesetzt, wobei Hal ein Halogenatom, vorzugsweise Chlor oder Brom bedeutet und X, Y und Z die unter Formel I angegebene Bedeutung haben.

Das 4-(5′-Halogenpyridyl-2′-oxy)-phenol wird durch Umsetzen von entsprechend substituierten 2,5-Dihalogenpyridinen mit Hydrochinon in an sich bekannter Weise erhalten.

Die Derivate der 2-Halogenpropionsäure werden in an sich bekannter Weise durch Reaktion von Hydroxycarbonsäure bzw. Mercaptocarbonsäure-Derivaten mit 2-Halogenpropionsäurehalogeniden erhalten.

b)

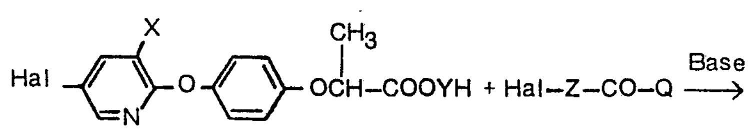


I

Die aus dem Stand der Technik bekannten O - [4 - (5′ - Halogenpyridyl - 2′ - oxy) - phenyl] - milchsäuren oder -thiolsäuren, siehe z.B. die DOS 2 546 251 oder 2 732 846, werden in an sich bekannter Weise in Gegenwart einer Base als säurebindendes Mittel mit Estern von Halogencarbonsäuren umgesetzt. Diese Ester entsprechen der Formel

$$Hal—Z—CO—Q$$

worin Hal ein Halogenatom, vorzugsweise Chlor oder Brom und Q und Z die unter Formel I gegebene Bedeutung haben und sind z.B. Ester, Thioester oder Amide der Chloressigsäure, der Bromessigsäure, der $\alpha$-Brompropionsäure, der $\alpha$-Brombuttersäure, der $\beta$-Chlor oder Brompropionsäure, der $\beta$-Brombuttersäure, der $\gamma$-Brombuttersäure etc.

c)

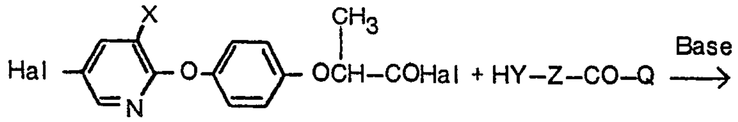


I

In dieser Modifikation zum obenstehenden Verfahren werden Säurehalogenide der O - [4 - (5' - Halogenpyridyl - 2' - oxy) - phenyl] - milchsäuren in an sich bekannter Weise mit Estern von Hydroxy- oder Merkaptocarbonsäuren umgesetzt. Beispiele solcher Säuren sind die Milchsäure die Glykolsäure, die β-Hydroxypropionsäure, β- oder γ-hydroxy-buttersäuren wie auch die entsprechenden Thiolsäuren, Thioglykolsäure, Thiomilchsäure etc.

Die Umsetzungen werden vorzugsweise in einem gegenüber den Reaktionskomponenten inerten Lösungsmittel durchgeführt. Als Lösungsmittel kommen solche aus den verschiedensten Stoffklassen in Frage, wie aliphatische und aromatische, gegebenenfalls chlorierte Kohlenwasserstoffe, z.B. Aethylenchlorid etc., sowie polare organische Lösungsmittel, wie Aether, Ketone, Amide, stabile Ester, z.B. Methyläthylketon, Dimethoxyäthan, Dimethylformamid, Dimethylsulfoxid, Tetrahydrofuran etc.

Als basische Säureakzeptoren für die Umsetzung mit Halogenverbindungen können wässerige Alkalimetallhydroxide, wie KOH und NaOH sowie weitere übliche basische Stoffe, wie Karbonate ($K_2CO_3$, $NaHCO_3$), Alkoholate ($NaOCH_3$ und Kalium-tert.butylat), aber auch organische Basen wie Triäthylamin etc. verwendet werden.

Die z.B. im Schema b) als Ausgangsmaterial zu benutzende freie Propionthiolsäure (Y = S), sowie deren Herstellung aus dem entsprechenden Propionsäurehalogenid mit Schwefelwasserstoff, $Na_2S$ oder NaHS in Gegenwart eines basischen Säureakzeptors ist Gegenstand einer hängigen Patentanmeldung. Die nach dieser Methode hergestellte α - [4 - (3',5' - Dichlor - pyridyl - (2') - oxy) - phenoxy] - propionthiolsäure ist ein Oel vom Brechungs-index $n_D^{21} = 1,5787$, das nach Kristallisation bei 85—87°C schmilzt.

Die nachfolgenden Beispiele veranschaulichen die Herstellung einiger erfindungsgemässer Wirkstoffe der Formel I. Weitere in entsprechender Weise oder nach einer anderen der im Text erwähnten Methoden hergestellte Endstoffe der Formel I sind anschliessend tabellarisch aufgeführt.

## Beispiel 1

O-[4-(3',5'-Dichlorpyridyl-2'-oxy)-phenyl]-thiolmilchsäuremethoxycarbonylmethyl)-ester

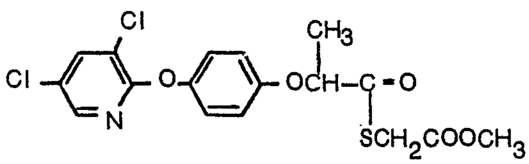

Zu 17,3 g (0,05 Mol) O - [4 - (3',5' - Dichlorpyridyl - 2' - oxy) - phenyl] - milchsäurechlorid in 130 ml Toluol werden 5,8 g (0,055 Mol) Thiolglycolsäuremethylester und 5,5 g (0,055 Mol) Triäthylamin gegeben. Nach dem Abklingen der Exothermie wird das Gemisch 2 Std. auf 40°C geheizt. Dann wird die Reaktion gekühlt, das Salz abfiltriert, das Filtrat eingedampft und in Chloroform über eine kurze Kieselgelsäule filtriert. Nach dem Eindampfen erhält man 13,5 g (64,8% der Theorie) des Titelproduktes mit dem Brechungsindex $n_D^{25}$ 1.5580 als Oel, das nach der Umkristallisation aus Aether/Petroläther bei 65°C schmilzt.

Das als Ausgangsmaterial benötigte Säurechlorid wurde die folgt erhalten:

26,8 g (0,082 M) O - [4 - (3',5' - Dichlorpyridyl - 2' - oxy) - phenyl] - milchsäure werden mit 30 ml Thionylchlorid versetzt und, nachdem die Gasentwicklung abgeklungen ist, auf 50°C erwärmt. Nach 2 Std. wird das Reaktionsgemisch am Vacuum eingedampft, mit 100 ml Toluol versetzt und wieder eingedampft. Als Produkt erhält man ein dunkelbraunes Oel, das langsam zu kristallisieren beginnt. Es werden 25,9 g (86,7% der Theorie) O - [4 - (3',5' - Dichlorpyridyl - 2' - oxy) - phenyl] - milchsäurechlorid vom Schmelzpunkt 45°C erhalten.

## Beispiel 2

O-[4-(3',5-Dichlorpyridyl-2'-oxy)-phenyl]-thiomilchsäure-(1''-methoxycarbonyl-äthyl)-ester

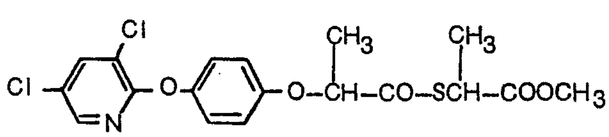

12,0 g (0,035 M) O - [4 - (3',5' - Dichloropyridyl - 2' - oxy) - phenyl] - thiomilchsäure werden in 50 ml Methyläthylketon gelöst und bei Raumtemperatur mit 5,5 g (0,04 M) Kaliumcarbonat versetzt. Dabei steigt die Temperatur unter Gasentwicklung auf 32°C. Nach 10 Minuten gibt man 4,25 ml

(0,038 M) 2-Brom-propionsäure-methylester zu. Die Reaktion ist exotherm und die Temperatur steigt auf 45°C. Nach 10 Minuten filtriert man das Reaktionsgemisch ab und erhält nach dem Eindampfen und Trocknen am Hochvacuum bei 55°C reines Titelprodukt, das einen Brechungsindex $n_D^{22} = 1{,}5779$ hat. Ausbeute: 13,8 g (91,4% der Theorie).

Die als Ausgangsmaterial benötigte Propionthiolsäure wurde wie folgt hergestellt.

17,2 g (0,0496 Mol) O - (4 - (3′,5′ - Dichlorpyridyl - 2′ - oxy) - phenyl) - milchsäurechlorid, hergestellt wie in Beispiel 1, werden einem Gemisch von 8,9 g KOH in 4,9 ml Wasser und 75 ml Dimethoxyäthan zugetropft, das bei 10—15°C unter starkem Rühren mit $H_2S$ gesättigt wurde. Während des Zutropfens hält man die Temperatur mit einem Eisbad bei 10°C. Anschliessend lässt man 30 min. bei Raumtemperatur ausrühren und giesst das Reaktionsgemisch auf 150 ml Eis/Wasser. Die trübe, braune Lösung wird mit conc. HCl auf pH 1 gestellt. Dabei fällt ein braunes Oel aus, das in Methylenchlorid aufgenommen wird. Die organische Phase wird direkt auf eine kleine Kieselgelsäule gegeben und mit Methylenchlorid durchgespült. Nach dem Eindampfen der hellgelben Lösung erhält man ein oranges, klares Oel. Dieses kristallisiert beim Verreiben mit Petroläther. Man erhält 16,8 g $\alpha$ - (4 - (3′,5′ - Dichlorpyridyl - 2′ - oxy) - phenoxy) - propionthiolsäure als gelbe Kristalle vom Schmelzpunkt 85—87°C.

Beispiel 3

O-[4-(3′,5′-Dichlorpyridyl-2′-oxy)-phenyl]-milchsäure-(1″-äthoxycarbonyläthyl)-ester

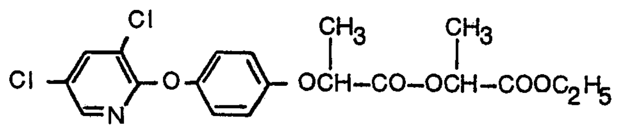

12,8 g (0,05 M) 4 - (3′,5′ - Dichlorpyridyl - 2′ - oxy) - phenol werden mit 8,3 g (0,06 M) Kaliumcarbonat in 50 ml Methyläthylketon eine Stunde zum Sieden erhitzt. Dann gibt man bei 50°C 13,2 g (0,052 M) O - (2′ - Bromopropionyl) - milchsäureäthylester zu und rührt das Reaktionsgemisch 12 Stunden bei 55°C. Die braune Suspension wird nun abfiltriert und das Filtrat am Rotationsverdampfer eingedampft. Das braune Oel chromatographiert man mit Petroläther über eine kleine Kieselgelsäule und erhält 10,9 g (52,7% der Theorie) vom Titelprodukt mit dem Brechungsindex $n_D^{22}$ 1,5424

Der als Zwischenprodukt verwendete O - (2′ - Brompropionyl) - milchsäure - äthylester der Formel

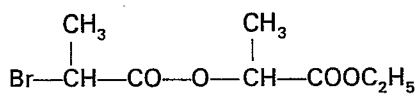

wurde wie folgt erhalten:

Zu 110 g (0,51 M) 2-Brompropionsäurebromid in 250 ml Toluol tropft man bei 10—15°C ein Gemisch von 60,2 g (0,51 M) Milchsäureäthylester und 51,5 g (0,51 M) Triäthylamin. Die dabei entstandene, weisse Suspension filtriert man nach 15 Minuten ab und dampft des Filtrat ein. Dein Rückstand destilliert man am Vacuum und erhält bei 78°C/0,6 mm 93,6 g (72,5% der Theorie) des Esters als klares Oel.

In analoger Weise zu diesen Beispielen wurden die in den folgenden Tabelle aufgeführten Ester hergestellt.

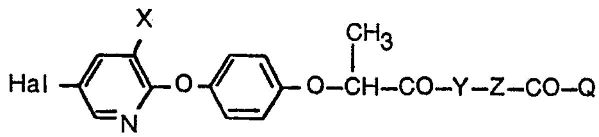

| No. | Hal | X | Y | Z | Q | Physikalische Konstante |
|---|---|---|---|---|---|---|
| 1 | Cl | Cl | S | $CH_2$ | $OCH_3$ | $n_D^{25}$ 1.5580 |
| 2 | Cl | Cl | S | $CH(CH_3)$ | $OCH_3$ | $n_D^{22}$ 1.5779 |
| 3 | Cl | Cl | O | $CH(CH_3)$ | $OC_2H_5$ | $n_D^{22}$ 1.5424 |
| 4 | Cl | Cl | S | $CH(CH_3)$ | $NHC_2H_5$ | Smp. 113–14 °C |
| 5 | Cl | Cl | O | $CH(CH_3)$ | $NHC_2H_5$ | |
| 6 | Cl | Cl | S | $CH_2$ | $NH_2$ | Smp. 98–100 °C |
| 7 | Cl | Cl | O | $CH_2$ | $NH_2$ | Smp. 154–59 °C |
| 8 | Cl | Cl | O | $C_2H_4$ | $NH_2$ | |
| 9 | Cl | Cl | S | $C_2H_4$ | $OCH_3$ | |
| 10 | Cl | Cl | O | $C_2H_4$ | $OCH_3$ | |
| 11 | Cl | Cl | O | $CH_2$ | $OCH_3$ | $n_D^{22}$ 1.5489 |
| 12 | Cl | Cl | O | $CH_2$ | OH | |
| 13 | Cl | Cl | S | $CH_2$ | OH | |
| 14 | Cl | Cl | O | CHCN | $OC_2H_5$ | |
| 15 | Cl | Cl | O | $CHCOOC_2H_5$ | $OC_2H_5$ | $n_D^{22}$ 1.5339 |
| 16 | Cl | Cl | S | $CHCOOC_2H_5$ | $OC_2H_5$ | $n_D^{22}$ 1.5461 |
| 17 | Cl | Cl | S | CHCN | $OC_2H_5$ | $n_D^{22}$ 1.5612 |
| 18 | Cl | Cl | O | $CHCOCH_3$ | $OCH_3$ | |
| 19 | Cl | Cl | S | $CHCOCH_3$ | $OCH_3$ | |
| 20 | Cl | H | S | $CH_2$ | $OCH_3$ | |
| 21 | Cl | H | O | $CH_2$ | $OCH_3$ | |
| 22 | Cl | Cl | S | $OC_2H_5$ | | $n_D^{22}$ 1.5598 |
| 23 | Cl | Cl | S | $C_2H_4$ | $OC_2H_5$ | $n_D^{22}$ 1.5580 |
| 24 | Cl | Cl | O | $CHCOCH_3$ | $OC_2H_5$ | $n_D^{22}$ 1.5253 |
| 25 | Cl | Cl | S | $C_2H_4$ | $NH_2$ | Smp. 109–10 °C |

Die neuen Wirkstoffe der Formel I sind stabile Verbindungen, welche in üblichen organischen Lösungsmitteln, wie Alkoholen, Aethern, Ketonen, Dimethylformamid, Dimethylsulfoxyd etc. löslich sind.

Die Erfindung betrifft auch herbizide und pflanzenwachstumsregulierende Mittel, welche einen neuen Wirkstoff der Formel I enthalten, wie Verfahren zur pre- und post-emergenten Unkrautbekämpfung und zur Hemmung des Pflanzenwachstums von monocotylen und dicotylen Pflanzen, insbesondere Gräsern, Getreide, Soja und Tabakgeiztrieben.

Die erfindungsgemässen Mittel können in den üblichen Formulierungen vorliegen.

Die Herstellung erfindungsgemässer Mittel erfolgt in an sich bekannter Weise durch inniges Vermischen und Vermahlen von Wirkstoffen der Formel I mit geeigneten Trägerstoffen, gegebenenfalls

unter Zusatz von gegenüber den Wirkstoffen inerten Dispersions- oder Lösungsmitteln. Die Wirkstoffe können in den folgenden Aufarbeitungsformen vorliegen und angewendet werden:

| | |
|---|---|
| feste Aufarbeitungsformen: | Stäubemittel, Streumittel, Granulate, Umhüllungsranulate Imprägnierungsgranulate und Homogengranulate; |
| in Wasser diespergierbare Wirkstoffkonzentrate: | Spritzpulver, (wettable powder), Pasten, Emulsionen: |
| flüssige Aufarbeitungsformen: | Lösungen. |

Die Wirkstoffkonzentrationen betragen in den erfindungsgemässen Mitteln 1 bis 80 Gewichtsprozent und können gegebenenfalls bei der Anwendung auch in geringen Konzentrationen wie etwa 0,05 bis 1% vorliegen.

Den beschriebenen erfindungsgemässen Mitteln lassen sich andere biozide Wirkstoffe oder Mittel beimischen. So können die neuen ausser den geannten Verbindungen der allgemeinen Formel I z.B. Insektizide Fungizide, Bakterizide, Fungistatika, Bakteriostatika. Nematozide oder weitere Herbizide zur Verbreiterung des Wirkungsspektrums enthalten.

Zur Veranschaulichung der Herstellung erfindungsgemässer Mittel sind einige Beispiele von festen und flüssigen Aufarbeitungsformen beschrieben.

*Spritzpulver*

Zur Herstellung eines a) 50%igen, b) 25%igen und c) 10%igen Spritzpulvers werden folgende Bestanteile verwendet:

| | | | |
|---|---|---|---|
| a) | 50 | Teile | Wirkstoff, |
| | 5 | Teile | Natriumdibutylnaphthylsulfonat, |
| | 3 | Teile | Naphthalinsulfonsäuren — Phenolsulfonsäuren-Formaldehyd-Kondensat 3:2:1, |
| | 20 | Teile | Kaolin, |
| | 22 | Teile | Champagne-Kreide; |
| b) | 25 | Teile | Wirkstoff, |
| | 5 | Teile | Oleylmethyltaurid-Natrium-Salz, |
| | 2,5 | Teile | Naphthalinsulfonsäuren-Formaldehyd-Kondensat, |
| | 0,5 | Teile | Carboxymethylcellulose, |
| | 5 | Teile | neutrales Kalium-Aluminium-Silikat, |
| | 62 | Teile | Kaolin; |
| c) | 10 | Teile | Wirkstoff, |
| | 3 | Teile | Gemisch der Natriumsalze von gesättigten Fettalkoholen, |
| | 5 | Teile | Naphthalinsulfonsäuren-Formaldehyd-Kondensat, |
| | 82 | Teile | Kaolin. |

Der angegebene Wirkstoff wird auf die entsprechenden Trägerstoffe (Kaolin und Kreide) aufgezogen und anschliessend mit den übrigen Bestandteilen vermischt und vermahlen. Man erhält Spritzpulver von vorzüglicher Benetzbarkeit und Schwebenfähigkeit. Aus solchen Spritzpulvern können durch Verdünnen mit Wasser Suspensionen mit einer Wirkstoffkonzentration erhalten werden, die sich zur Unkrautbekämpfung in Pflanzenkulturen eignen.

*Paste*

Zur Herstellung eine 45%igen Paste werden folgende Stoffe verwendet:

| | | |
|---|---|---|
| 45 | Teile | eines der Wirkstoffe der Formel I |
| 5 | Teile | Natriumaluminiumsilikat, |
| 14 | Teile | Cetylpolyäthylenglykoläther mit 8 Mol Aethylenoxid, |
| 1 | Teil | Oleylpolyäthylenglykoläther mit 5 Mol Aethylenoxid, |
| 2 | Teile | Spindeloel, |
| 10 | Teile | Polyäthenglykol, |
| 23 | Teile | Wasser. |

Der Wirkstoff wird mit den Zuschlagstoffen in dazu geeigneten Geräten innig vermischt und vermahlen. Man erhält eine Past, aus der sich durch Verdünnen mit Wasser Suspensionen jeder gewünschten Konzentration herstellen lassen.

*Emulsionskonzentrat*

Zur Herstellung eines 25%igen Emulsionskonzentrates werden

| | | |
|---|---|---|
| 25 | Teile | eines der Wirkstoffe der Formel I, |
| 5 | Teile | Mischung von Nonylphenolpolyoxyäthylen und Calciumdodecylbenzolsulfat, |
| 15 | Teile | Cyclohexanon, |
| 55 | Teile | Xylol |

mit einander vermischt. Dieses Konzentrat kann mit Wasser zu Emulsionen auf geeigneten Konzentrationen von z.B. 0,1 bis 10% verdünnt werden. Solche Emulsionen eignen sich zur Bekämpfung von Unkräutern in Kulturpflanzungen.

Erfindungsgemässe Mittel, die als aktive Komponente mindestens eine Verbindung der Formel I enthalten, eignen sich besonders zur selektiven Bekämpfung monocotyler Ungräser in pre- und insbesondere post-emergenter Anwendung in Kulturpflanzenbeständen, wie z.B. Soja, Baumwolle, Zuckerrüben, Weizen und anderen Gereidearten, etc.

Zum Nachweis der Brauchbarkeit als Herbizide (pre- und post-emergent) und zum Beweis der Ueberlegenheit gegenüber bekannten Wirkstoffen ähnlicher Strucktur dienen folgende Testmethoden:

*Pre-emergente Herbizid-Wirkung* (Keimhemmung)

Im Gewächshaus wird unmittelbar nach der Einsaat der Versuchspflanzen in Saatschalen die Erdoberfläche mit einer wässerigen Dispersion der Wirkstoffe, erhalten aus einem 25%-igen Emulsionskonzentrat resp. aus einem 25%-igen Spritzpulver mit Wirkstoffen, die wegen ungenügender Löslichkeit nicht als Emulsionskonzentrat hergestellt werden können, behandelt. Es wurden zwei verschiedene Konzentrationsreihen angewendet, entsprechend 4 und 2 kg Wirksubstanz pro Hektar. Die Saatschalen werden im Gewächshaus bei 22—25°C und 50—70% rel. Luftfeuchtigkeit gehalten und der Versuch nach 3 Wochen ausgewertet und die Resultate nach folgender Notenskala bonitiert:

1 = Pflanzen nicht gekeimt oder total abgestorben
2—3 = sehr starke Wirkung
4—6 = mittlere Wirkung

7—8 = geringe Wirkung
9 = keine Wirkung (wie unbehandelte Kontrolle)

In diesem Test werden erfindungsgemässe O - (Pyridyloxy-phenoxy) - milchsäureester und folgende strukturähnliche Verbindungen verglichen:

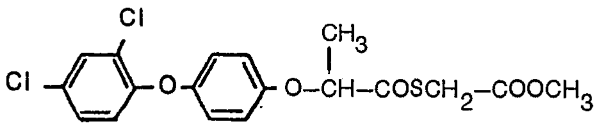

A

O - [4 - (2' - 4' - Dichlorphenoxy) - phenyl - thio - milchsäure(methoxycarbonylmethyl) - ester, bekannt aus der DOS 2 628 384, Beispiel 3

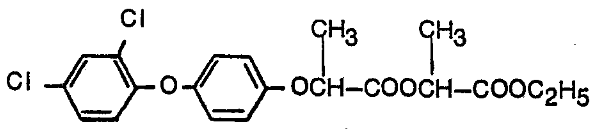

B

O - [4 - (2',4' - Dichlorphenoxy) - phenyl - milchsäure - (äthoxycarbonyläthyl) - ester, bekannt aus der DOS 2 628 384, Beispiel 23.

Es wurden folgende Pflanzen geprüft: Gerste, Baumwolle, sowie die Unkräuter avena fatua, alopercurus myosuroides, rottboellia exaltata und digitiaria sanguinalis.

Die Resultate sind in der untenstehenden Tabelle zusammengefasst.

| Verbindung No. | 1 | | 2 | | 3 | | 4 | | 6 | | 7 | | 11 | | 15 | | 16 | | 17 | | 22 | | 23 | | A | | B | |
|---|---|---|---|---|---|---|---|---|---|---|---|---|---|---|---|---|---|---|---|---|---|---|---|---|---|---|---|---|
| Aufwandmenge kg/ha | 4 | 2 | 4 | 2 | 4 | 2 | 4 | 2 | 4 | 2 | 4 | 2 | 4 | 2 | 4 | 2 | 4 | 2 | 4 | 2 | 4 | 2 | 4 | 2 | 4 | 2 | 4 | 2 |
| Gerste | 9 | 9 | 9 | 9 | 9 | 9 | 9 | 9 | 9 | 9 | 9 | 9 | 9 | 9 | 7 | 9 | 9 | 9 | 9 | 9 | 6 | 9 | 9 | 9 | 9 | 9 | 9 | 9 |
| Baumwolle | 9 | 9 | 9 | 9 | 9 | 9 | 9 | 9 | 9 | 9 | 9 | 9 | 9 | 9 | 9 | 9 | 9 | 9 | 9 | 9 | 9 | 9 | 9 | 9 | 9 | 9 | 9 | 9 |
| avena fatua | 2 | 3 | 4 | 4 | 3 | 3 | 2 | 9 | 2 | 2 | 4 | 4 | 3 | 6 | 3 | 3 | 3 | 3 | 6 | 6 | 2 | 7 | 3 | 3 | 8 | 8 | 9 | 9 |
| alopecurus myosuroides | 1 | 1 | 2 | 2 | 1 | 4 | 1 | 2 | 1 | 1 | 2 | 6 | 1 | 2 | 1 | 1 | 2 | 2 | 2 | 2 | 1 | 2 | 1 | 1 | 6 | 6 | 7 | 9 |
| rottboellia exaltata | 1 | 1 | 2 | 4 | 1 | 1 | 1 | 1 | 1 | 1 | 1 | 2 | 1 | 1 | 1 | 1 | 1 | 1 | 1 | 2 | 1 | 1 | 1 | 1 | 3 | 4 | 7 | 8 |
| digitaria sanguinalis | 1 | 1 | 1 | 1 | 1 | 1 | 1 | 1 | 1 | 1 | 1 | 1 | 1 | 1 | 1 | 1 | 1 | 2 | 1 | 1 | 1 | 1 | 1 | 1 | 1 | 1 | 1 | 2 |

*Post-emergente Herbizid-Wirkung (Kontaktherbizid)*

Eine grössere Anzahl (mindestens 7) Unkräuter und Kulturpflanzen, sowohl monocotyle wie dicotyle, wurden nach dem Auflaufen (im 4-bis-6-Blattstadium) mit einer wässerigen Wirkstoffdisperion in Dosierungen von 1 und 1/2 kg Wirksubstanz pro Hektar auf die Pflanzen gespritzt und diese bei 24°—26°C und 45—60% rel. Luftfeuchtigkeit gehalten. 15 Tage nach Behandlung wird der Versuch ausgewertet und das Ergebnis wie im pre-emergent-Versuch nach derselben Notenskala bonitiert.

Es wurden folgende Pflanzen geprüft: Weizen, Soja, Zuckerrübe, sowie die Unkräuter avena fatua, lolium perenne, alopecurus myosuroides und setaria italica.

Die Resultate sind in der untenstehenden Tabelle zusammengefasst.

| Verbindung No. | 1 | | 2 | | 3 | | 4 | | 6 | | 7 | | 11 | | 15 | | 16 | | 17 | | 22 | | 23 | | 24 | | A | | B | |
|---|---|---|---|---|---|---|---|---|---|---|---|---|---|---|---|---|---|---|---|---|---|---|---|---|---|---|---|---|---|---|
| Aufwandmenge kg/ha | 1 | 0.5 | 1 | 0.5 | 1 | 0.5 | 1 | 0.5 | 1 | 0.5 | 1 | 0.5 | 1 | 0.5 | 1 | 0.5 | 1 | 0.5 | 1 | 0.5 | 1 | 0.5 | 1 | 0.5 | 1 | 0.5 | 1 | 0.5 | 1 | 0.5 |
| Weizen | 8 | 9 | 7 | 9 | 1 | 8 | 7 | 9 | 1 | 5 | 8 | 9 | 6 | 9 | 1 | 9 | 4 | 9 | 1 | 2 | 9 | 9 | 5 | 9 | 9 | 9 | 9 | 9 | 9 | 9 |
| Soja | 9 | 9 | 9 | 9 | 9 | 9 | 9 | 9 | 9 | 9 | 9 | 9 | 9 | 9 | 9 | 9 | 9 | 9 | 9 | 9 | 9 | 9 | 9 | 9 | 9 | 9 | 8 | 9 | 9 | 9 |
| Zuckerrübe | 9 | 9 | 9 | 9 | 9 | 9 | 9 | 9 | 9 | 9 | 9 | 9 | 9 | 9 | 9 | 9 | 9 | 9 | 9 | 9 | 9 | 9 | 9 | 9 | 9 | 9 | 9 | 9 | 9 | 9 |
| avena fatua | 1 | 2 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 4 | 5 | 9 | 9 |
| lolium perenne | 1 | 1 | 1 | 1 | 1 | 1 | 1 | 1 | 1 | 1 | 1 | 1 | 1 | 1 | 1 | 1 | 1 | 1 | 1 | 1 | 1 | 2 | 1 | 1 | 1 | 2 | 2 | 2 | 6 | 7 |
| alopecurus myosuroides | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 7 | 7 | 9 | 9 |
| setaria italica | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 2 | 4 | 9 |

**Patentansprüche für die Vertragsstaaten: BE, CH, DE, FR, GB, IT und NL**

1. O-(Pyridyloxy-phenyl)-milchsäure-ester der Formel I

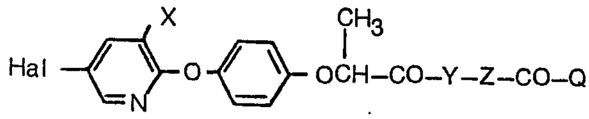 I

worin

Hal ein Halogenatom,

X Wasserstoff oder ein Halogenatom,

Y Sauerstoff oder Schwefelatom,

Z eine $C_1$—$C_4$ Alkylengruppe, die gegebenenfalls durch Methyl, Aethyl, Cyan, Methylcarbonyl oder eine Gruppe —COQ substituiert sein kann,

Q ein Rest —$OR_1$, —$SR_2$ oder —$NR_3R_4$,

$R_1$ und $R_2$ unabhängig voneinander je Wasserstoff oder das Kation-äquivalent einer

$$\frac{1}{n}\text{Base—}M^{n\oplus},$$

wobei

M ein n-wertiges Alkali- oder Erdalkalimetall Kation oder ein Fe-, Cu-, Zn-, Mn-, Ni-Kation oder einen Ammonio-Rest

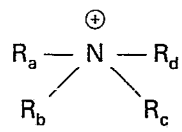

n als ganze Zahl 1, 2 oder 3 der Wertigkeit des Kations entspricht, während $R_a$, $R_b$, $R_c$ und $R_d$ unabhängig voneinander Wasserstoff, Benzyl oder einen gegebenenfalls durch —OH, —$NH_2$ oder $C_1$—$C_4$ Alkoxy substituierten $C_1$—$C_4$ Alkylrest bedeuten.

— einen $C_1$—$C_{18}$ Alkyl-Rest, der unsubstituiert oder gegebenenfalls substituiert ist durch Halogen, Nitro, Cyano, $C_1$—$C_8$ Alkoxy, $C_2$—$C_8$ Alkoxy-alkoxy; $C_3$—$C_6$ Alkenyloxy, $C_1$—$C_8$ Alkylthio, $C_2$—$C_8$ Alkanoyl, $C_2$—$C_8$ Acyloxy, $C_2$—$C_8$ Alkoxycarbonyl, Carbamoyl; Bis ($C_1$—$C_4$ alkyl) amino, Tris-($C_1$—$C_4$ alkyl)ammonio, $C_3$—$C_8$ Cycloalkenyl, gegebenenfalls auch durch einen unsubstituierten oder seinerseits durch Halogen, $C_1$—$C_4$ Alkyl, $C_1$—$C_4$ Alkoxy ein- oder mehrfach substituierten Phenyl-, Phenoxy- oder 5-6-gliedrigen heterocyclischen Rest mit 1 bis 3 Heteroatomen;

— einen unsubst. oder ein- bis vierfach durch Halogen oder einen durch Phenyl oder Methoxycarbonyl subst. $C_3$—$C_{18}$ Alkenylrest

— einen $C_3$—$C_8$ Alkinyl-Rest

— einen gegebenenfalls durch Halogen oder $C_1$—$C_4$ Alkyl substituierten $C_3$—$C_{12}$ Cycloalkyl-Rest;

— einen $C_3$—$C_8$ Cycloalkenyl-Rest;

— einen Phenylrest, der unsubstituiert oder durch Halogen, $C_1$—$C_4$-Alkyl, $C_1$—$C_4$-Alkoxy, $C_1$—$C_4$-Alkylthio, $NO_2$, $CF_3$, COOH, CN, OH, $SO_3H$, $NH_2$ oder —NH($C_1$—$C_4$ Alkyl) oder —N($C_1$—$C_4$ Alkyl)$_2$ ein- oder mehrfach substituiert ist;

— einen 5- bis 6-gliedrigen heterocyclischen Ring mit 1 bis 3 Heteroatomen

$R_3$ und $R_4$ unabhängig voneinander je Wasserstoff oder $C_1$—$C_4$ Alkyl, das gegebenenfalls durch Chlor, Cyan, Hydroxyl oder $C_1$—$C_4$ Alkoxy substituiert ist

$C_3$—$C_4$ Alkenyl

$R_3$ zudem auch $C_3$—$C_4$ Alkinyl, $C_1$—$C_4$ Alkoxy, Phenyl oder Benzyl, oder

$R_3$ und $R_4$ zusammen mit dem Stickstoffatom, an das sie gebunden sind auch einen 5-6 gliedrigen Heterocyclus bilden, der auch Sauerstoff, Schwefel oder eine Gruppe

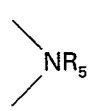

enthalten kann,

$R_5$ Wasserstoff, $C_1$—$C_4$ Alkyl oder Phenyl bedeuten.

2. Als Verbindung gemäss Anspruch 1 O - [4 - (3′ - 5′ - Dichlorpyridyl - 2′ - oxy) - phenyl] - thiomilchsäure - (methoxycarbonylmethyl) - ester.

3. Als Verbindung gemäss Anspruch 1 O - [4 - (3′,5′ - Dichlorpyridyl - 2′ - oxy) - phenyl] - thiomilchsäure - [methoxycarbonyläthyl) - ester.

4. Verfahren zur Herstellung der O - (Pyridyloxy - phenyl) - milchsäure - ester der Formel I, Anspruch 1, dadurch gekennzeichnet, dass man in an sich bekannter Weise gemäss den untenstehenden Schemata

a)

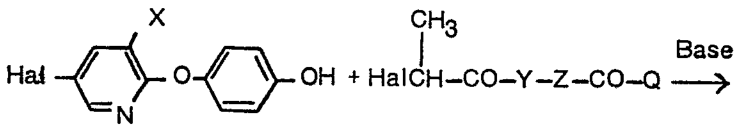


I

ein entsprechend substituiertes 4 - (5' - Halogenpyridyl - 2' - oxy) - phenol in Gegenwart einer Base mit einem 2-Halogenpropion-säure-Derivat der Formel

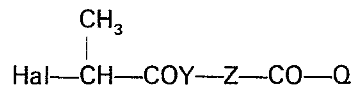

umsetzt, wobei Hal, Q, X, Y und Z die unter Formel I, Anspruch 1 gegebene Bedeutung haben;

b)

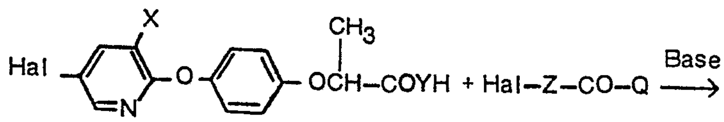


I

eine O - [4 - (5' - Halogenpyridyl - 2' - oxy) - phenyl] - milchsäure oder -thiomilchsäure in Gegenwart einer Base mit einem Halogencarbonsäureester der Formel

Hal—Z—CO—Q

umsetzt, wobei Hal, Q, X, Y und Z die unter Formel I, Anspruch 1 gegebene Bedeutung haben, oder

c)

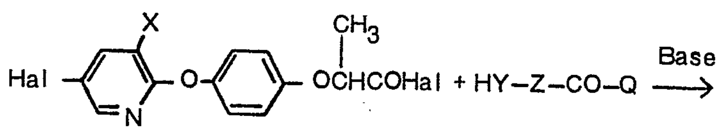


I

ein Säurehalogenid einer O - [4 - (5' - Halogenpyridyl - 2' - oxy) - phenyl] - milchsäure in Gegenwarteiner Base mit einem Ester einer Hydroxycarbonsäure oder Merkaptocarbonsäure der Formel

HY—Z—CO—Q

umsetzt, wobei Hal, Q, X, Y und Z die unter Formel I, Anspruch 1 gegebene Bedeutung haben.

5. Herbizides und das Pflanzenwachstum regulierendes Mittel, dadurch gekennzeichnet, dass es als Wirkstoff mindestens einen O - (Pyridyloxy - phenyl) - milchsäureester der Formel I, Anspruch 1 enthält.

6. Die Verwendung der O - (Pyridyloxy - phenyl) - milchsäureester der Formel I, Anspruch 1, oder sie enthaltender Mittel als Herbizide.

7. Die Verwendung der O - (Pyridyloxy - phenyl) - milchsäureester der Formel I, Anspruch 1, oder sie enthaltender Mittel zur Regulierung des Pflanzenwachstums.

**Claims for the Contracting States: BE, CH, DE, FR, GB, IT, NL**

1. O-(Pyridyloxy-phenyl)-lactic acid ester of the formula I

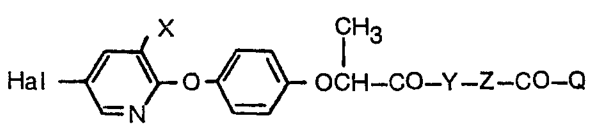

(I)

in which

"Hal" is a halogen atom,

X is hydrogen or a halogen atom,

Y is an oxygen or sulfur atom,

Z is a $C_1$—$C_4$-alkylene group which is unsubstituted or substituted by methyl, ethyl, cyano, methylcarbonyl or by a group —COQ,

Q is a group —$OR_1$, —$SR_2$ or —$NR_3R_4$, wherein

$R_1$ and $R_2$ independently of one another are each hydrogen or the cation equivalent of a

$$\text{base} \overset{1}{\underset{n}{—}} M^{n\oplus} ,$$

wherein M is an n-valent alkali metal cation or alkaline earth metal cation, or an Fe, Cu, Zn, Mn or Ni cation, or an ammonium group

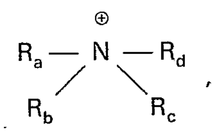

n as an integer 1, 2 or 3 corresponds to the valency of the cation, whilst $R_a$, $R_b$, $R_c$ and $R_d$ independently of one another were ech hydrogen, benzyl, or a $C_1$—$C_4$-alkyl group which is unsubstituted or substituted by —OH, —$NH_2$ or $C_1$—$C_4$-alkoxy;

or $R_1$ and $R_2$ independently of one another are each $C_1$—$C_{18}$-alkyl group which is unsubstituted or is substituted by halogen, nitro, cyano, $C_1$—$C_8$-alkoxy, $C_2$—$C_8$-alkoxy-alkoxy, $C_3$—$C_6$-alkenyloxy, $C_1$—$C_8$-alkylthio, $C_2$—$C_8$-alkanoyl, $C_2$—$C_8$-acyloxy, $C_2$—$C_8$-alkoxycarbonyl, carbamoyl, bis-($C_1$—$C_4$-alkyl)-amino, tris-($C_1$—$C_4$-alkyl)-ammonium, $C_3$—$C_8$-cycloalkenyl, or by a phenyl, phenoxy or 5-6-membered heterocyclic radical having 1 to 3 hetero atoms, each of which is unsubstituted or for its part is mono- or polysubstituted by halogen, $C_1$—$C_4$-alkyl or $C_1$—$C_4$-alkoxy; or they are each a $C_3$—$C_{18}$-alkenyl group which is unsubstituted or is mono- or tetrasubstituted by halogen or is monosubstituted by phenyl or methoxycarbonyl; or they are a $C_3$—$C_8$-alkynyl group; or a $C_3$—$C_{12}$-cycloalkyl group which is unsubstituted or is substituted by halogen or $C_1$—$C_4$-alkyl; or they are each a $C_3$—$C_8$-cycloalkenyl group; or a phenyl group which is unsubstituted or is mono- or polysubstituted by halogen, $C_1$—$C_4$-alkyl, $C_1$—$C_4$-alkoxy, $C_1$—$C_4$-alkylthio, $NO_2$, $CF_3$, COOH, CN, OH, $SO_3H$, $NH_2$ or —NH($C_1$—$C_4$-alkyl) or —N($C_1$—$C_4$-alkyl)$_2$; or $R_1$ and $R_2$ are each a 5- to 6-membered heterocyclic ring having 1 to 3 hetero atoms, and

$R_3$ and $R_4$ independently of one another are each hydrogen, or $C_1$—$C_4$-alkyl which is unsubstituted or is substituted by chlorine, cyano, hydroxyl or $C_1$—$C_4$-alkoxy, or they are each $C_3$—$C_4$-alkenyl and

$R_3$ is also $C_3$—$C_4$-alkynyl, $C_1$—$C_4$-alkoxy, phenyl or benzyl, or

$R_3$ and $R_4$ together with the nitrogen atom to which they are attached also form a 5-6-membered heterocycle which can contain oxygen, sulfur or a group

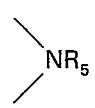

wherein $R_5$ is hydrogen, $C_1$—$C_4$-alkyl or phenyl.

2. As a compound according to Claim 1: O - [4 - (3′,5′ - dichloropyridyl - 2′ - oxy) - phenyl] - thiolactic acid-(methoxycarbonylmethyl) ester.

3. As a compound according to Claim 1: O - [4 - (3′,5′ - dichloropyridyl - 2′ - oxy) - phenyl] - thiolactic acid-(methoxycarbonylethyl) ester.

4. A process for producing the O - (pyridyloxy - phenyl) - lactic acid ester of the formula I, Claim 1, which process comprises reacting in a manner known per se, according to the reaction patterns given below,

a)

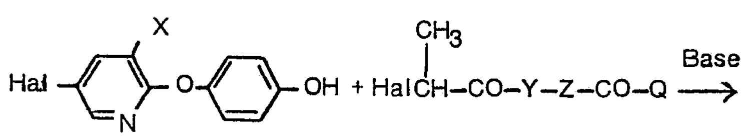


I

an appropriately substituted 4 - (5′ - halogenopyridyl - 2′ - oxy) - phenol, in the presence of a base, with a 2-halogenopropionic acid derivatives of the formula

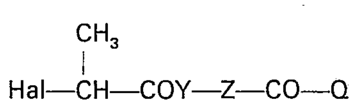

wherein "Hal", Q, X, Y and Z have the meanings defined under the formula I, Claim 1;

b)

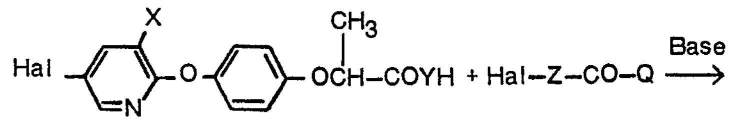


I

reacting an O - [4 - (5' - halogenopyridyl - 2' - oxy) - phenyl] - lactic acid or -thiolactic acid, in the presence of a base, with a halogenocarboxylic acid ester of the formula

Hal—Z—CO—Q,

wherein "Hal", Q, X, Y and Z have the meanings defined under the formula I, Claim 1; or

c)

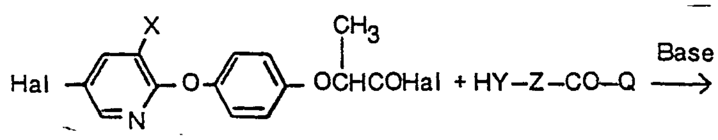


I

reacting an acid halide or an O - [4 - (5' - halogenopyridyl - 2' - oxy) - phenyl] - lactic acid, in the presence of a base, with an ester of a hydroxycarboxylic acid or a mercaptocarboxylic acid of the formula

HY—Z—CO—Q,

wherein "Hal", Q, X, Y and Z have the meanings defined under the formula I, Claim 1.

5. A herbicidal and plant-growth-regulating composition which contains as active substance at least one O - (pyridyloxy - phenyl) - lactic acid ester of the formula I, Claim 1.

6. The use of the O - (pyridyloxy - phenyl) - lactic acid esters of the formula I, Claim 1, or of compositions containing them, as herbicides.

7. The use of the O - (pyridyloxy - phenyl) - lactic acid esters of the formula I, Claim 1, or of compositions containing them, for regulating plant growth.

**Revendications pour les Etats contractants: BE, CH, DE, FR, GB, IT, NL**

1. Esters O-(pyridyloxy-phényl)-lactiques de formule I:

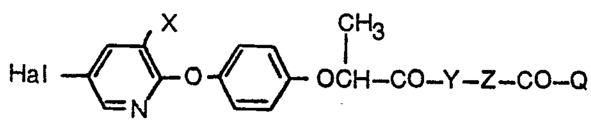

(I)

dans laquelle Hal représente un atome d'halogène,

X représente l'hydrogène ou un atome d'halogène,

Y représente un atome d'oxygène ou de soufre

Z représente un groupe alkylène en C1—C4 qui peut éventuellement être substitué par des groupes méthyle, éthyle, cyano, méthylcarbonyle ou un groupe COQ,

Q représente un groupe $—OR_1$, $—SR_2$ ou $—NR_3R_4$,

$R_1$ et $R_2$ représentent chacun, indépendamment l'un de l'autre, l'hydrogène ou l'équivalent d'un cation d'une

$$\text{base} \frac{1}{n} M^{n\oplus},$$

M représente un cation de métal alcalin ou alcalino-terreux ou un cation Fe, Cu, Zn, Mn, Ni, de valence n, ou un reste ammonio

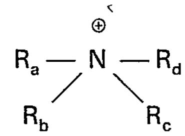

n, nombre entier égal à 1, 2 ou 3, correspond à la valence du cation et $R_a$, $R_b$, $R_c$ et $R_d$ représentent chacun, indépendamment les uns des autres, l'hydrogène, un groupe benzyle ou un groupe alkyle en C1—C4 portant éventuellement des substituants —OH, —$NH_2$ ou alcoxy en C1—C4,

— un groupe alkyle en C1—C18, non substitué ou éventuellement substitué par des halogènes, des groupes nitro, cyano, alcoxy en C1—C8, alcoxy-alcoxy en C2—C8; alcenyloxy en C3—C6, alkythio en C1—C8, alcanoyle en C2—C8, acyloxy en C2—C8, alkoxycarbonyle en C2—C8, carbamoyle; bis-(alkyle en C1—C4)-amino, tris-(alkyle en C1—C4)-ammonio cycloalcényle en C3—C8, le cas échéant également par un groupe phényle, phénoxy ou un reste hétérocyclique de 5 à 6 chaînons contenant 1 à 3 hétéroatomes, non substitué ou lui-même mono-ou poly- substitué par des halogènes, des groupes alkyles en C1—C4, alcoxy en C1—C4;

— un reste alcényle en C3—C18 non substitué ou mono- à tétra-substitué par des halogènes ou un reste alcényle en C3—C18 portant un substituant phényle ou méthoxycarbonyle,

— un reste alcynyle en C3—C8,

— un reste cycloalkyle en C3—C12 éventuellement substitué par des halogènes ou des groupes alkyles en C1—C4;

— un reste cycloalcényle en C3—C8;

— un reste phényle non substitué ou mono- ou poly-substitué par des halogènes, des groupes alkyles en C1—C4, alcoxy en C1—C4, alkylthio en C1—C4, $NO_2$, $CF_3$, COOH, CN, OH, $SO_3H$, $NH_2$ ou —NH(alkyle en C1—C4) ou —N(alkyle en $C1—C4)_2$;

— un noyau hétérocyclique de 5 à 6 chaînons contenant 1 à 3 hétéroatomes, $R_3$ et $R_4$ représentent chacun, indépendamment l'un de l'autre l'hydrogène ou un groupe alkyle en C1—C4 éventuellement substitué par le chlore, des groupes cyano, hydroxy ou alcoxy en C1—C4, un groupe alcényle en C3—C4,

$R_3$ pouvant en outre représenter un groupe alcynyle en C3—C4, alcoxy en C1—C4, phényle ou benzyle, ou bien

$R_3$ et $R_4$ forment ensemble et avec l'atome d'azote auquel ils sont reliés un noyau hétérocyclique de 5 à 6 chaînons qui peut contenir également de l'oxygène, du soufre ou un groupe

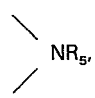

$R_5$ représente l'hydrogène, un groupe alkyle en C1—C4 ou phényle.

2. En tent que composé selon la revendication 1, l'O - [4 - (3',5' - dichloropyridyl - 2' - oxy) - phenyl] - thiolactate de méthoxycarbonylméthyle.

3. En tant que composé selon la revendication 1, l'O - [4 - (3',5' - dichloropyridyl - 2' - oxy) - phényl] - thiolactate de méthoxycarbonyléthyle.

4. Procédé de préparation des esters O-(pyridyloxyphényl)-lactiques de formule I, revendication 1, caractérisé en ce que l'on fait réagir de manière connue en soi, selon les schémes ci-après:

a)

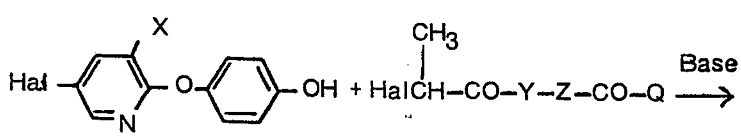


I

un 4 - (5' - halogénopyridyl - 2' - oxy) - phénol portant les substituants correspondants en présence d'une base avec un dérivé d'acide 2-halogéno-propionique de formule:

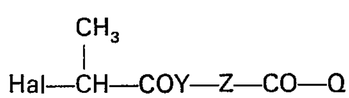

dans laquelle Hal, Q, X, Y et Z ont les significations indiquées en référence à la formule I, revendication 1;

b)

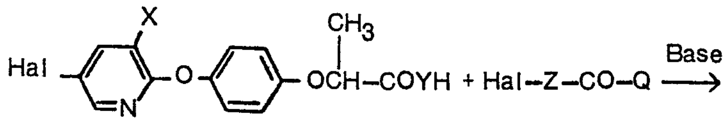

I

on fait réagir un acid O - [4 - (5' - halogénopyridyl - 2' - oxy) - phényl] - lactique ou -thiolactique en présence d'une base avec un ester d'acide halogénocarboxylique de formule:

Hal—Z—CO—Q

dans laquelle Hal, Q, X, Y et Z ont les significations indiquées en référence à la formule I, revendication 1, ou bien:

c)

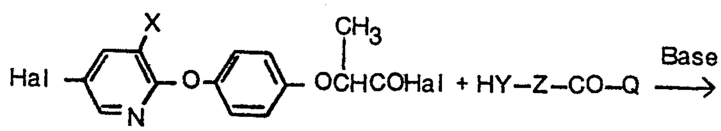

I

on fait réagir un halogénure d'un acide O - [4 - (5' - halogénopyridyl - 2' - oxy) - phényl] - lactique en présence d'une base avec un ester d'un acide hydroxycarboxylique ou mercaptocarboxylique de formule:

HY—Z—CO—Q

dans laquelle Hal, Q, X, Y et Z ont les significations indiquées en référence à la formule I, revendication 1.

5. Produit herbicide et régulateur de la croissance des végétaux caractérisé en ce qu'il contient en tant que substance active au moins un ester O-(pyridyloxyphényl)-lactique de formule I, revendication 1.

6. L'utilisation des esters O-(pyridyloxy-phényl)-lactiques de formule I, revendication 1, ou de produits en contenant en tant qu'herbicides.

7. L'utilisation des esters O-(pyridyloxy-phényl)-lactiques de formule I, revendication 1, ou de produits en contenant pour la régulation de la croissance de végétaux.

**Patentansprüche für den Vertragsstaat: AT**

1. Herbizides und das Pflanzenwachstum regulierendes Mittel dadurch gekennzeichnet, dass es als Wirkstoff einen O-Pyridyloxy-phenyl)-milchsäureester der Formel I, enthält.

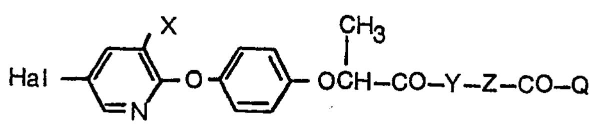
(I)

worin
Hal ein Halogenatom,
X Wasserstoff oder ein Halogenatom,
Y Sauerstoff- oder Schwefelatom
Z eine $C_1$—$C_4$ Alkylengruppe, die gegebenenfalls durch Methyl, Aethyl, Cyan, Methylcarbonyl oder eine Gruppe —COQ substituiert sein kann,
Q ein Rest —$OR_1$, —$SR_2$ oder —$NR_3R_4$,
$R_1$ und $R_2$ unabhängig voneinander je Wasserstoff oder das Kation-äquivalent einer

$$\frac{1}{n}\text{Base—}M^{n\oplus},$$

wobei
M ein n-wertiges Alkali- oder Erdalkalimetall-Kation oder ein Fe-, Cu-, Zn-, Zn-, Mn-, Ni-Kation oder einen Ammonio-Rest

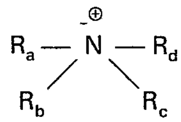

n als ganze Zahl 1, 2 oder 3 der Wertigkeit des Kations entspricht, während $R_a$, $R_b$, $R_c$ und $R_d$ unabhängig voneinander Wasserstoff, Benzyl oder einen gegebenenfalls durch —OH, —$NH_2$ oder $C_1$—$C_4$ Alkoxy substituierten $C_1$—$C_4$ Alkylrest bedeuten.

— einen $C_1$—$C_{18}$ Alkyl-Rest, der unsubstituiert oder gegebenenfalls substituiert ist durch Halogen, Nitro, Cyano, $C_1$—$C_8$ Alkoxy, $C_2$—$C_8$ Alkoxy-alkoxy; $C_3$—$C_6$ Alkenyloxy, $C_1$—$C_8$ Alkylthio, $C_2$—$C_8$ Alkanoyl, $C_2$—$C_8$ Acyloxy, $C_2$—$C_8$ Alkoxycarbonyl, Carbamoyl; Bis ($C_1$—$C_4$ alkyl) amino, Tris-($C_1$—$C_4$ alkyl)ammonio, $C_3$—$C_8$ Cycloalkenyl, gegebenenfalls auch durch einen unsubstituierten oder seinerseits durch Halogen, $C_1$—$C_4$ Alkyl, $C_1$—$C_4$ Alkoxy ein- oder mehrfach substituierten Phenyl-, Phenoxy- oder 5,6-gliedrigen heterocyclischen Rest mit 1 bis 3 Heteroatom;

— einen unsubst. oder ein- bis vierfach durch Halogen oder einen durch Phenyl oder Methoxycarbonyl subst. $C_3$—$C_{18}$ Alkenylrest

— einen $C_3$—$C_8$ Alkinyl-Rest

— einen gegebenenfalls durch Halogen oder $C_1$—$C_4$ Alkyl substituierten $C_3$—$C_{12}$ Cycloalkyl-Rest;

— einen $C_3$—$C_8$ Cycloalkenyl-Rest;

— einen Phenylrest, der unsubstituiert oder durch Halogen, $C_1$—$C_4$-Alkyl, $C_1$—$C_4$-Alkoxy, $C_1$—$C_4$-Alkylthio, $NO_2$, $CF_3$, COOH, CN, OH, $SO_3H$, $NH_2$ oder —NH($C_1$—$C_4$ Alkyl) oder —N($C_1$—$C_4$ Alkyl)$_2$ ein- oder mehrfach substituiert ist;

— einen 5- bis 6-gliedrigen heterocyclischen Ring mit 1 bis 3 Heteroatomen

$R_3$ und $R_4$ unabhängig voneinander je Wasserstoff oder $C_1$—$C_4$ Alkyl das gegebenenfalls durch Chlor, Cyan, Hydroxyl oder $C_1$—$C_4$ Alkoxy substituiert ist,

$C_3$—$C_4$ Alkenyl

$R_3$ zudem auch $C_3$—$C_4$ Alkinyl, $C_1$—$C_4$ Alkoxy, Phenyl oder Benzyl, oder

$R_3$ und $R_4$ zusammen mit dem Stickstoffatom, an das sie gebunden sind auch einen 5—6 gliedrigen Heterocyclus bilden, der auch Sauerstoff, Schwefel oder eine Gruppe

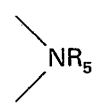

enthalten kann,

$R_5$ Wasserstoff, $C_1$—$C_4$ Alkyl oder Phenyl bedeuten, zusammen mit einem Trägerstoff und anderen inerten Zutaten.

2. Verfahren zur Herstellung der O - (Pyridyloxy - phenyl) - milchsäure - ester der Formel I, Anspruch 1, dadurch gekennzeichnet, dass man in an sich bekannter Weise gemäss den untenstehenden Schemata

a)

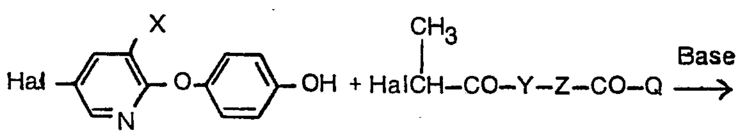


I

ein entsprechend substituiertes 4 - (5′ - Halogenpyridyl - 2′ - oxy) - phenol in Gegenwart einer Base mit einem 2-Halogenpropion-säure-Derivat der Formel

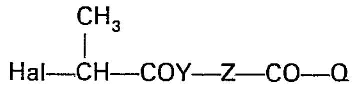

umsetzt, wobei Hal, Q, X, Y und Z die unter Formel I, Anspruch 1 gegebene Bedeutung haben;

b)

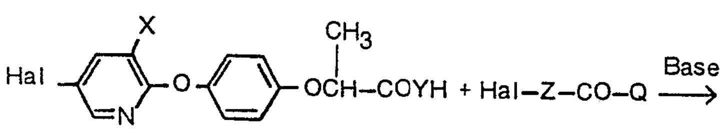

eine O - [4 - (5′ - Halogenpyridyl - 2′ - oxy) - phenyl] - milchsäure oder -thiomilchsäure in Gegenwart einer Base mit einem Halogencarbonsäureester der Formel

Hal—Z—CO—Q

umsetzt, wobei Hal, Q, X, Y und Z die unter Formel I, Anspruch I gegebene Bedeutung haben, oder

c)

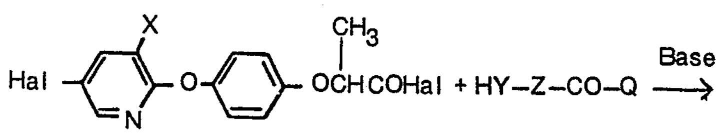


I

ein Säurehalogenid einer O - [4 - (5′ - Halogenpyridyl - 2′ - oxy) - phenyl] - milchsäure in Gegenwarteiner Base mit einem Ester einer Hydroxycarbonsäure oder Merkaptocarbonsäure der Formel

HY—Z—CO—Q

umsetzt, wobei Hal, Q, X, Y und Z die unter Formel I, Anspruch 1 gegebene Bedeutung haben.

3. Die Verwendung der O - (Pyridyloxy - phenyl) - milchsäure - ester der Formel I, Anspruch 1, oder sie enthaltender Mittel zur selektiven Bekämpfung von Unkräutern in Nutzpflanzenkulturen.

4. Die Verwendung gemäss Anspruch 3 von O - [4 - (3′,5′ - Dichlorpyridyl - 2′ - oxy) - phenyl] - thiomilchsäure - (methoxycarbonylmethyl) - ester.

5. Die Verwendung gemäss Anspruch 3 von O - [4 - (3′,5′ - Dichlorpyridyl - 2′ - oxy) - phenyl] - thiomilchsäure - (methoxycarbonyl - äthyl) - ester.

**Claims for the Contracting State: AT**

1. A herbicidal and plant-growth-regulating composition, characterised in that it contains as active ingredient an O-(pyridyloxy-phenyl)-lactic acid ester of the formula I

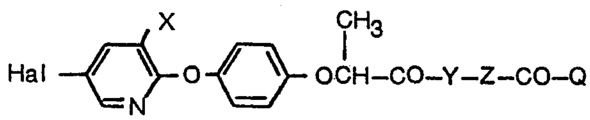

(I)

in which

"Hal" is a halogen atom,

X is hydrogen or a halogen atom,

Y is an oxygen or sulfur atom,

Z is a $C_1$—$C_4$-alkylene group which is unsubstituted or substituted by methyl, ethyl, cyano, methylcarbonyl or by a group —COQ,

Q is a group —$OR_1$, —$SR_2$ or —$NR_3R_4$, wherein

$R_1$ and $R_2$ independently of one another are each hydrogen or the cation equivalent of a

$$\text{base} \frac{1}{n} M^{n\oplus}$$

wherein M is an n-valent alkali metal cation or alkaline earth metal cation, or an Fe, Cu, Zn, Mn or Ni cation, or an ammonium group

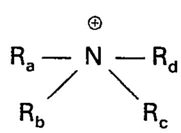

n as an integer 1, 2 or 3 corresponds to the valency of the cation, whilst $R_a$, $R_b$, $R_c$ and $R_d$ independently of one another were each hydrogen, benzyl, or a $C_1$—$C_4$-alkyl group which is unsubstituted or substituted by —OH, —$NH_2$ or $C_2$—$C_4$-alkoxy;

or $R_1$ and $R_2$ independently of one another are each $C_1$—$C_{18}$-alkyl group which is unsubstituted or is substituted by halogen, nitro, cyano, $C_1$—$C_8$-alkoxy $C_2$—$C_8$-alkoxy-alkoxy, $C_3$—$C_6$-alkenyloxy, $C_1$—$C_8$-alkylthio, $C_2$—$C_8$-alkanoyl, $C_2$—$C_8$-acyloxy, $C_2$—$C_8$-alkoxycarbonyl, carbamoyl, bis-($C_1$—$C_4$-alkyl)-amino, tris-($C_1$—$C_4$-alkyl)-ammonium, $C_3$—$C_8$-cycloalkenyl, or by a phenyl, phenoxy or 5-6-membered

heterocyclic radical having 1 to 3 hetero atoms, each of which is unsubstituted or for its part is mono- or polysubstituted by halogen, $C_1$—$C_4$-alkyl or $C_1$—$C_4$-alkoxy; or they are each a $C_3$—$C_{18}$-alkenyl group which is unsubstituted or is mono- or tetrasubstituted by halogen or is monosubstituted by phenyl or methoxycarbonyl; or they are a $C_3$—$C_8$-alkynyl group; or a $C_3$—$C_{12}$-cycloalkyl group which is unsubstituted or is substituted by halogen or $C_1$—$C_4$-alkyl or they are each a $C_3$—$C_8$-cycloalkenyl group; or a phenyl group which is unsubstituted or is mono- or polysubstituted by halogen, $C_1$—$C_4$-alkyl, $C_1$—$C_4$-alkoxy, $C_1$—$C_4$-alkylthio, $NO_2$, $CF_3$, COOH, CN, OH, $SO_3H$, $NH_2$ or —NH($C_1$—$C_4$-alkyl) or —N($C_1$—$C_4$-alkyl)$_2$; or $R_1$ and $R_2$ are each a 5- to 6-membered heterocyclic ring having 1 to 3 hetero atoms, and

$R_3$ and $R_4$ independently of one another are each hydrogen, or $C_1$—$C_4$-alkyl which is unsubstituted or is substituted by chlorine, cyano, hydroxyl or $C_1$—$C_4$-alkoxy, or they are each $C_3$—$C_4$-alkenyl and

$R_3$ is also $C_3$—$C_4$-alkynyl, $C_1$—$C_4$-alkoxy, phenyl or benzyl, or

$R_3$ and $R_4$ together with the nitrogen atom to which they are attached also form a 5-6-membered heterocycle which can contain oxygen, sulfur or a group

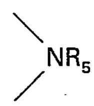

wherein $R_5$ is hydrogen, $C_1$—$C_4$-alkyl or phenyl, together with a carrier and other inert ingredients.

2. A process for producing the O-(pyridyloxy-phenyl)-lactic acid ester of the formula I, Claim 1, which process comprises reacting in a manner known per se, according to the reaction patterns given below,

a)

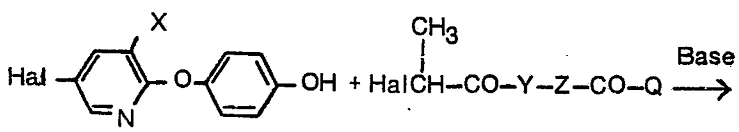


I

an appropriately substituted 4 - (5′ - halogenopyridyl - 2′ - oxy) - phenol, in the presence of a base, with a 2-halogenopropionic acid derivative of the formula

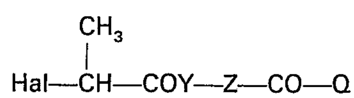

wherein "Hal", Q, X, Y and Z have the meanings defined under the formula I, Claim 1;

b)

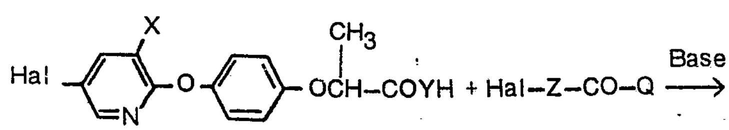


I

reacting an O - [4 - (5′ - halogenopyridyl - 2′ - oxy) - phenyl] - lactic acid or -thiolactic acid, in the presence of a base, with a halogenocarboxylic acid ester of the formula

Hal—Z—CO—Q,

wherein "Hal", Q, X, Y and Z have the meanings defined under the formula I, Claim 1; or

c)

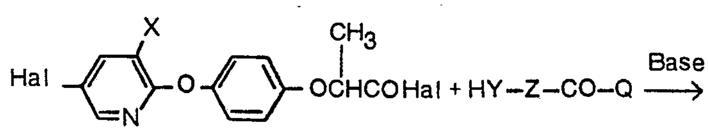


I

reacting an acid halide or an O - [4 - (5′ - halogenopyridyl - 2′ - oxy) - phenyl] - lactic acid, in the presence of a base, with an ester of a hydroxycarboxylic acid or a mercaptocarboxylic acid of the formula

HY—Z—CO—Q,

wherein "Hal", Q, X, Y and Z have the meanings defined under the formula I, Claim 1.

3. A method of selectively combating gramineous weeds in crops of cultivated plants, which method comprises applying thereto or to the locus thereof an effective amount of an O-(pyridyloxy-phenyl)-lactic acid ester of the formula I, Claim 1, or of a composition containing it.

4. A method according to Claim 3, which method comprises applying an effective amount of O - [4 - (3',5' - dichloropyridyl - 2' - oxy) - phenyl] - thiolactic acid-(methoxycarbonylmethyl) ester.

5. A method according to Claim 3, which method comprises applying an effective amount of O - [4 - (3',5' - dichloropyridyl - 2' - oxy) - phenyl] - thiolactic acid-(methoxycarbonylethyl) ester.

**Revendications pour L'Etat contractant: AT**

1. Produit herbicide et régulateur de la croissance des végétaux caractérisé en ce qu'il contient en tant que substance active un ester O-(pyridyloxy-phényl)-lactique de formule I:

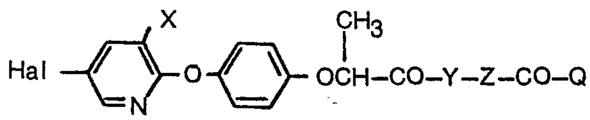

(I)

dans laquelle Hal représente un atome d'halogène,

X représente l'hydrogène ou un atome d'halogène,

Y représente un atome d'oxygène ou de soufre

Z représente un groupe alkylène en C1—C4 qui peut éventuellement être substitué par des groupes méthyle, éthyle, cyano, méthylcarbonyle ou un groupe COQ,

Q représente un groupe $—OR_1$, $—SR_2$ ou $—NR_3R_4$,

$R_1$ et $R_2$ représentent chacun, indépendamment l'un de l'autre, l'hydrogène ou l'équivalent d'un cation d'une

$$\text{base} — \frac{1}{n} M^{n\oplus},$$

M représente un cation de métal alcalin ou alcalino-terreux ou un cation Fe, Cu, Zn, Mn, Ni, de valence n, ou un reste ammonio

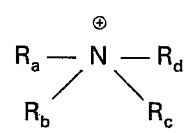

n, nombre entier égal à 1, 2 ou 3, correspond à la valence du cation et $R_a$, $R_b$, $R_c$ et $R_d$ représentent chacun, indépendamment les uns des autres, l'hydrogène, un groupe benzyle ou un groupe alkyle en C1—C4 portant éventuellement des substituants —OH, $—NH_2$ ou alcoxy en C1—C4,

— un groupe alkyle en C1—C18, non substitué ou éventuellement substitué par des halogènes, des groupes nitro, cyano, alcoxy en C1—C8, alcoxy-alcoxy en C2—C8; alcenyloxy en C3—C6, alkythio en C1—C8, alcanoyle en C2—C8, acyloxy en C2—C8, alkoxycarbonyle en C2—C8, carbamoyle; bis-(alkyle en C1—C4)-amino, tris-(alkyle en C1—C4)-ammonio cycloalcényle en C3—C8, le cas échéant également par un groupe phényle, phénoxy ou un reste hétérocyclique de 5 à 6 chaînons contenant 1 à 3 hétéroatomes, non substitué ou lui-même mono-ou poly- substitué par des halogènes, des groupes alkyles en C1—C4, alcoxy en C1—C4;

— un reste alcényle en C3—C18 non substitué ou mono- à tétra-substitué par des halogènes ou un reste alcényle en C3—C18 portant un substituant phényle ou méthoxycarbonyle,

— un reste alcynyle en C3—C8,

— un reste cycloalkyle en C3—C12 eventuellement substitué par des halogènes ou des groupes alkyles en C1—C4;

— un reste cycloalcényle en C3—C8;

— un reste phényle non substitué ou mono- ou poly-substitué par des halogènes, des groupes

alkyles en C1—C4, alcoxy en C1—C4, alkylthio en C1—C4, $NO_2$, $CF_3$, COOH, CN, OH, $SO_3H$, $NH_2$ ou —NH(alkyle en C1—C4) ou —N(alkyle en C1—C4)$_2$;

— un noyau hétérocyclique de 5 à 6 chaînons contenant 1 à 3 hétéroatomes, $R_3$ et $R_4$ représentent chacun, indépendemment l'un de l'autre l'hydrogène ou un groupe alkyle en C1—C4 éventuellement substitué par le chlore, des groupes cyano, hydroxy ou alcoxy en C1—C4, un groupe alcényle en C3—C4,

$R_3$ pouvant en outre représenter un groupe alcynyle en C3—C4, alcoxy en C1—C4, phényle ou benzyle, ou bien

$R_3$ et $R_4$ forment ensemble et avec l'atome d'azote auquel ils sont reliés un noyau hétérocyclique de 5 à 6 chaînons qui peut contenir également de l'oxygène, du soufre ou un groupe

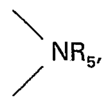

$R_5$ représente l'hydrogène, un groupe alkyle en C1—C4 ou phényle, avec un véhicule et d'autres additifs inertes.

2. Procédé de préparation des esters O - (pyridyloxy - phényl) - lactiques de formule I, revendication 1, caractérisé en ce que l'on fait réagir de manière connue en soi, selon les schémas ci-après:

a)

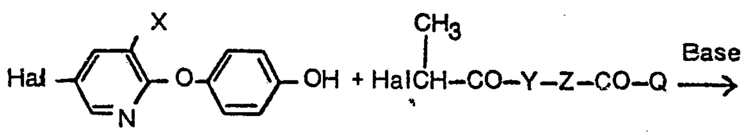


I

un 4 - (5' - halogénopyridyl - 2' - oxy) - phénol portant les substituants correspondants en présence d'une base avec un dérivé d'acide 2-halogéno-propionique de formule:

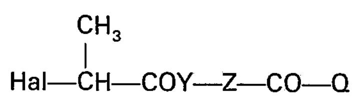

dans laquelle Hal, Q, X, Y et Z ont les significations indiquées en référence à la formule I, revendication 1;

b)

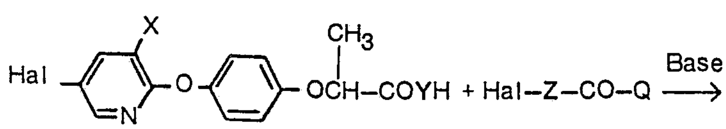


I

on fait réagir un acid O - [4 - (5' - halogénopyridyl - 2' - oxy) - phényl] - lactique ou-thiolactique en présence d'une base avec un ester d'acide halogénocarboxylique de formule:

Hal—Z—CO—Q

dans laquelle Hal, Q, X, Y et Z ont les significations indiquées en référence à la formule I, revendication 1, ou bien:

c)

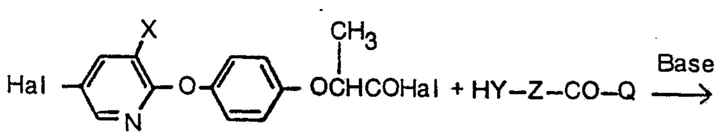


I

on fait réagir un halogénure d'un acide O - [4 - (5' - halogénopyridyl - 2' - oxy) - phényl] - lactique en présence d'une base avec un ester d'un acide hydroxycarboxylique ou mercaptocarboxylique de formule:

HY—Z—CO—Q

dans laquelle Hal, Q, X, Y et Z ont les significations indiquées en référence à la formule I, revendication 1.

3. L'utilisation des esters O-(pyridyloxy-phényl)-lactiques de formule I, revendication 1, ou de produits en contenant pour combattre sélectivement les mauvaises herbes du type graminées dans les cultures de végetaux utiles.

4. L'utilisation selon la revendication 1 de l'O - [4 - (3',5' - dichloropyridyl - 2' - oxy) - phényl] - thiolactate de méthoxycarbonylméthyle.

5. L'utilisation selon la revendication 3 de l'O - [4 - (3',5' - dichloropyridyl - 2' - oxy) - phényl] - thiolactate de méthoxycarbonyléthyle.